# FASCICULE DE BREVET EUROPEEN

(11) **EP 0 498 709 B1**
(45) Date de publication et mention de la délivrance du brevet: **25.10.1995**
(21) Numéro de dépôt: 92400275.1
(22) Date de dépôt: 03.02.1992
(51) Int. Cl.: A61B 17/58

(54) **Implant chirurgical osseux pour stabilisateur inter-vertèbral**
Implantat für Knochenchirurgie für Zwischenwirbelstabilisator
Implant for bone surgery for intervertebral stabliser

(30) Priorité: 05.02.1991 FR 9101288
(43) Date de publication de la demande: 12.08.1992
(73) Titulaire: SCIENCE ET MEDECINE (Société anonyme), F-75014 Paris (FR); Graf, Henry, F-69002 Lyon (FR)
(72) Inventeur: Graf, Henry, F-69002 Lyon (FR); Breard, Francis, Henri, F-75014 Paris (FR)

(56) Documents cités:
- EP-A- 0 260 970
- EP-A- 0 381 588
- FR-A- 1 240 313
- US-A- 4 869 242

## Description

La présente invention concerne un implant chirurgical pour stabilisateur inter-vertébral, formé d'une tige intra-osseuse qui, d'un côté, comporte une extrémité filetée d'implantation dans l'os et, de l'autre, se prolonge par une tête extra-osseuse cylindrique à section circulaire susceptible de soutenir au moins deux ligaments souples par l'une de leurs extrémités en forme de boucle.

Dans un stabilisateur inter-vertébral du type décrit dans la demande de brevet français No. 89 01445, de tels implants, en étant, par leur tige intra-osseuse, respectivement ancrés dans les vertèbres d'un tronçon à traiter du rachis, constituent deux à deux, par leur tête extra-osseuse, des points d'accrochage pour les extrémités d'un ligament souple respectif, ainsi retenu librement entre deux vertèbres successives.

De tels stabilisateurs présentent l'avantage de permettre la correction d'un grand nombre de défauts anatomiques du rachis, sans pour autant entraver les mouvements naturels de flexion et de torsion du tronc du patient.

Il s'avère toutefois que la répétition de ces mouvements peut, dans certains cas, conduire à une usure progressive des ligaments, due à un frottement excessif de chacun d'eux avec la surface rugueuse des vertèbres ou à un frottement mutuel des extrémités de deux ligaments retenues l'une au contact de l'autre sur la tête extra-osseuse d'un même implant. Une telle usure modifiant inévitablement la tension d'origine des ligaments touchés et étant même susceptible de provoquer leur rupture, elle impose au patient la contrainte d'une nouvelle intervention chirurgicale en vue de la réfection du stabilisateur inter-vertébral ainsi endommagé.

La présente invention vise à remédier à cet inconvénient et, pour ce faire, elle propose un implant chirurgical, du type spécifié en introduction, qui se caractérise en ce que la tête extra-osseuse présente au moins deux zones de retenue de ligament définies sur les surfaces périphériques de tronçons respectifs étagés de ladite tête extra-osseuse, chacune de ces zones de retenue étant délimitée, au moins à son extrémité la plus proche de la tige intra-osseuse, par un épaulement annulaire constituant une butée axiale pour ligament.

Sur la tête extra-osseuse de l'implant, un tel épaulement constitue une butée axiale pour un ligament respectif qui, étant ainsi confiné dans une zone de retenue, est à l'abri de tout frottement source d'usure, vis-à-vis de l'os et/ou d'un autre ligament.

C'est ainsi que selon l'une des possibilités offertes par l'invention, le ou l'un des épaulements est formé au voisinage de l'extrémité, contiguë à la tige, de la tête extra-osseuse, pour ainsi maintenir le ligament à l'écart de la surface de l'os.

De préférence, ledit épaulement est défini, au moins partiellement, par une collerette radiale, qui limite la pénétration dans l'os de la tige intra-osseuse et définit automatiquement l'écartement recherché entre le ligament et la surface de l'os.

Avantageusement, ladite collerette se prolonge sur une certaine hauteur le long de la tige, en direction de l'extrémité d'implantation de cette dernière. Lorsque l'implant est mis en place dans le fond d'une concavité de l'os, cette collerette de grande hauteur permet de rehausser la tête extra-osseuse pour ainsi éloigner le ligament retenu sur celle-ci des parois de la concavité et lui éviter tout frottement latéral avec celles-ci.

Selon une autre possibilité de l'invention, constituant un complément ou une variante à la première, le ou l'un des épaulement(s) délimite une zone périphérique de retenue de ligament définie sur un tronçon de la tête extra-osseuse, au-delà d'un second tronçon de cette dernière, qui, de son côté, peut avantageusement constituer une seconde zone périphérique de retenue de ligament, délimitée, à son extrémité proche de la première, par un second épaulement.

Ainsi la tête extra-osseuse peut retenir deux extrémités de ligament l'une au-dessus de l'autre, en les isolant de tout contact ou frottement mutuel qui pourrait être également source d'usure.

Dans un mode de réalisation simple de conception, les épaulements des deux zones de retenue ménagées sur une tête extra-osseuse monobloc, sont définies par une collerette radiale unique formée sur celle-ci.

Selon une autre caractéristique de l'invention, l'un au moins des tronçons de la tête extra-osseuse est forme par un insert enfilé et immobilisé axialement sur un axe solidaire de la tige. Grâce à un tel insert interchangeable, on peut, en cas de besoin, modifier le diamètre de l'une ou l'autre des zones de retenue de ligament.

Dans ce cas, le ou lesdits épaulements peuvent être définis, au moins partiellement, par un rebord radial de l'insert saillant au voisinage de l'extrémité de celui-ci, proche de l'autre tronçon de la tête extra-osseuse.

Sur une tête extra-osseuse monobloc ou réalisée à partir d'un ou de plusieurs inserts, les deux tronçons de la tête sur lesquels sont formées les zones de retenue étagées de ligament, seront généralement cylindriques, mais l'un d'eux au moins peut avantageusement présenter une forme tronconique, droite ou renversée, pour permettre au ligament qu'il retient de prendre une position inclinée par rapport à l'axe de la tête extra-osseuse, conformément à la courbure locale du rachis.

Dans un mode de réalisation préféré de l'invention, la ou l'une au moins des zones de retenue est formée par la surface latérale d'un insert enfilé, par un perçage central, sur un axe solidaire de la tige et reposant sur un gradin par une base bombée formée sous l'épaulement de ladite zone de retenue, le perçage central de l'insert étant forme de deux parties s'évasant chacune vers une face d'extrémité respective de l'insert, sur laquelle elle débouche, et communiquant entre elles par un orifice ayant un diamètre à peine supérieur à celui de l'axe sur lequel est enfilé l'insert.

Grâce à ces dispositions, l'insert peut librement pivoter et permet ainsi au ligament qu'il retient de s'orienter conformément aux mouvements de flexion du rachis.

Le même résultat avantageux sera obtenu dans un autre mode de réalisation de l'invention, dans lequel la tête extra-osseuse se termine par une rotule sur laquelle est emboîté, avec liberté de pivotement, un insert dont la surface périphérique délimite une zone de retenue respective de ligament entre deux épaulements formés par des rebords saillants des faces d'extrémité de l'insert.

Le col de la rotule, par lequel celle-ci se rattache à une platine de la tête extra-osseuse, pourra par ailleurs constituer une seconde zone de retenue de ligament, de préférence par l'intermédiaire d'un second insert en forme de U embrassant le col, la surface extérieure de ce second insert étant délimitée par des rebords d'arrêt pour un ligament.

Plusieurs modes de réalisation de l'implant chirurgical conforme à l'invention vont maintenant être décrits plus en détails, mais uniquement à titre d'exemples non-limitatifs, en référence aux dessins annexés, dans lesquels :
- la figure 1 illustre la conception générale d'un stabilisateur inter-vertébral ;
- la figure 2 est une vue latérale, à plus grande échelle, d'un stabilisateur inter-vertébral représenté dans sa configuration d'implantation, avec des implants ayant des têtes extra-osseuses conformes à un premier mode de réalisation de l'invention ;
- la figure 3 montre un perfectionnement à un tel implant ;
- les figures 4 à 7 représentent des variantes de réalisation des têtes extra-osseuses de ces implants ;
- les figures 8 à 10 en représentent d'autres variantes de réalisation, partiellement en coupe ;
- les figures 11 à 13 illustrent une tête d'implant conforme à un deuxième mode de réalisation de l'invention ; et
- les figures 14 et 15 en illustrent un troisième mode de réalisation.

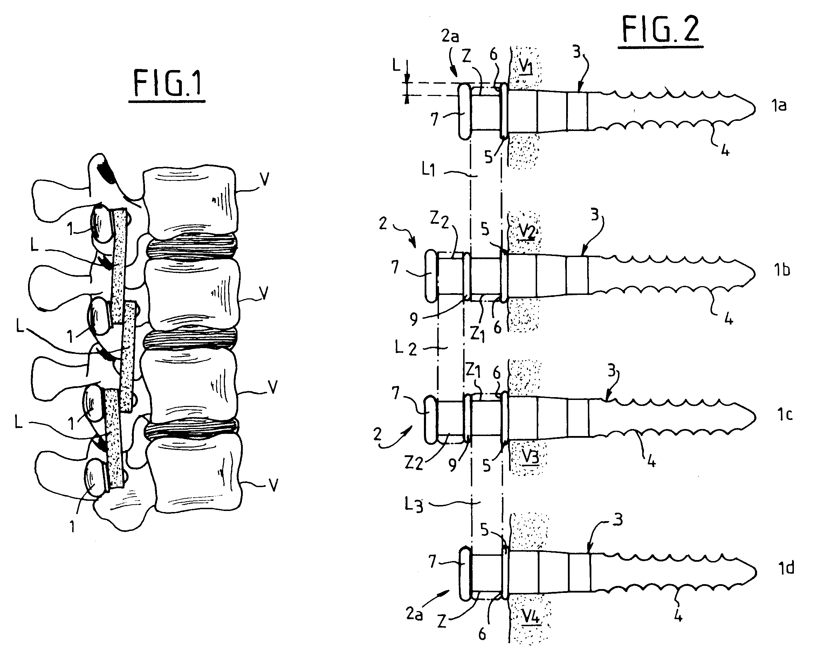

D'une manière générale, un stabilisateur inter-vertébral, comme le montre la figure 1, se compose de plusieurs ligaments artificiels souples L, montés en chaîne, à l'aide d'implants métalliques respectifs 1, au dos des vertèbres V constituant le tronçon de rachis traité.

Le stabilisateur représenté sur la figure 2 est realisé à l'aide de quatre implants 1a,1b,1c,1d conformes à l'invention destines à maintenir trois ligaments L₁, L₂, L₃, dessinés en traits mixtes, sur un tronçon de quatre vertèbres V₁, V₂, V₃, V₄. D'une manière connue en soi, chacun de ces ligaments présente la forme d'une boucle fermée et est retenu entre deux vertèbres successives, par ses extrémités engagées autour des têtes extra-osseuses 2,2a de deux implants respectivement ancrés dans ces vertèbres par une tige intra-osseuse 3 à bout fileté 4.

Sur chaque implant 1a à 1d, la tête extra-osseuse 2 ou 2a, ici cylindrique à section circulaire, surmonte la tige intra-osseuse 3 au-delà d'une collerette radiale 5 qui définit un épaulement annulaire 6 en regard de l'extrémité libre de la tête 2 ou 2a, quant à elle munie d'une coiffe circulaire amovible 7 surdimensionnée radialement. Cet épaulement 6 présente, dans la direction radiale, une largeur L comprise entre 1 et 3 fois l'épaisseur d'un ligament, ici conçu à l'aide d'une tresse plate de fils synthétiques.

Lors de l'ancrage d'un implant dans une vertèbre, la tige intra-osseuse 3 pénètre dans cette dernière jusqu'au contact de la collerette 5 avec la surface de l'os. L'épaulement 6 constitue alors, pour l'extrémité du ligament L₁, L₂ ou L₃ entourant la base de la tête 2 ou 2a, une butée la maintenant axialement à distance de l'os et lui évitant ainsi tout frottement, source d'usure et de rupture, avec celui-ci.

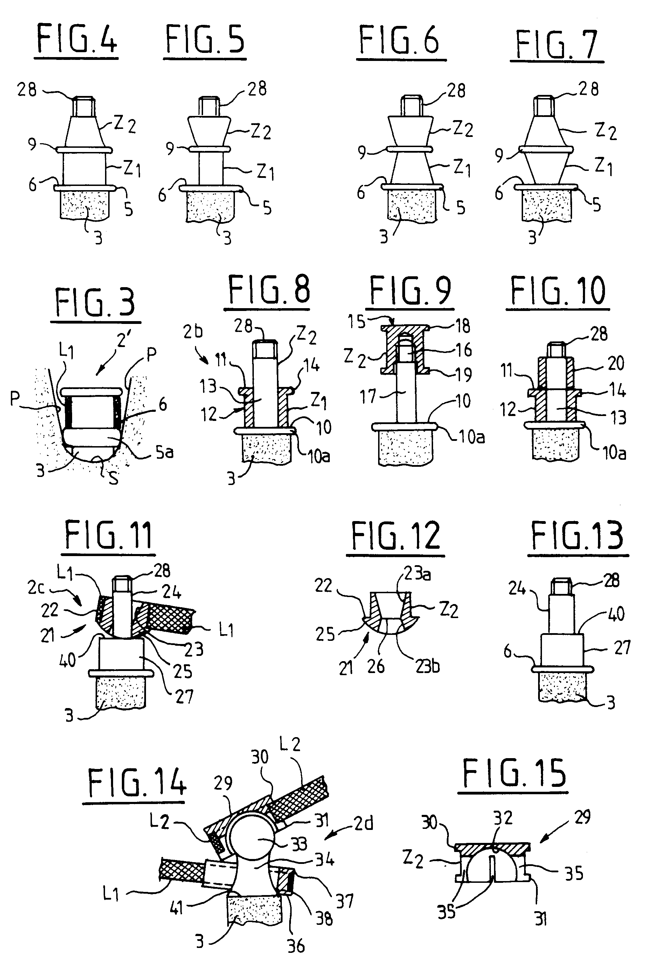

Comme représenté en 5a sur la figure 3, la hauteur de la collerette peut en outre être augmentée le long de la tige 3, pour surélever l'épaulement 6 par rapport à la surface S de l'os. Lorsque la tête 2′ de l'implant se trouve dans le fond d'une concavité de la vertèbre, l'épaulement 6 ainsi rehaussé maintient en plus le ligament L₁ à l'abri de tout frottement latéral avec les parois P de la concavité.

En revenant à la figure 2, on peut voir que sur chacun des implants d'extremité 1a et 1d du stabilisateur representé, l'épaulement 6 et la coiffe 7 délimitent, sur la surface cylindrique de la tête 2a, une zone de retenue unique Z, d'une hauteur sensiblement égale à la largeur d'un ligament, pour l'accrochage de l'extrémité respective du premier ou du troisième ligament L₁ ou L₃.

En revanche, la surface cylindrique de la tête allongée 2 de chacun des implants intermédiaires 1b et 1c, destinée à retenir, l'une au dessus de l'autre et selon des positions croisées, deux extrémités de ligament, est subdivisée entre l'épaulement 6 et la coiffe 7, en deux zones de retenue étagées Z₁,Z₂, de même hauteur que celle Z des deux autres implants, séparées par une collerette radiale 9 formée à mi-hauteur de la tête 2. Chacune des deux faces de cette collerette 9 constitue, pour le ligament accroché sur une zone de retenue respective Z₁ ou Z₂ de chaque tête 2, une butée le maintenant à l'écart du ligament portant sur l'autre zone de retenue Z₂ ou Z₁ et interdisant ainsi tout frottement mutuel qui serait là aussi susceptible d'occasionner une rupture de l'un ou l'autre des ligaments.

Les figures 4 à 7 montrent que l'une au moins des zones de retenue Z₁ et Z₂, séparées par la collerette 9, peut avoir une forme tronconique pour, en cas de besoin, permettre au ligament qu'elle supporte de prendre une certaine inclinaison par rapport à l'axe de la tête. Sur les figures 4 et 5, seule la zone supérieure Z₂ est formée sur un tronc de cône placé, en position droite ou respectivement renversée, sur le tronçon inférieur cylindrique de la tête 2 définissant la seconde zone de retenue Z₁. Sur les figures 6 et 7, les deux zones de retenue Z₁ et Z₂ sont formées sur des troncs de cône identiques joints par leur petite base ou respectivement leur grande base.

La figure 8 montre une tête extra-osseuse d'implant 2b formée à partir d'un insert cylindrique 12 qui est enfilé, par un perçage central, sur un axe 13 saillant sur une platine 10 de la tête, sur laquelle l'insert 12 repose, la platine 10 étant bordée d'une collerette 10a jouant le même rôle que la collerette 5 des têtes d'implant de la figure 2. Dans la tête ainsi constituée, la zone de retenue inférieure Z₁ est définie sur la surface latérale cylindrique de l'insert 12, entre un rebord supérieur radial 14 de ce dernier et l'épaulement défini par la platine 10, tandis que la zone de retenue supérieure Z₂ est formée sur le tronçon libre de l'axe 13, au-delà d'un gradin 11 constitué par la face supérieure de l'insert 12.

La figure 9 représente une variante selon laquelle l'insert 15 est vissé par un taraudage central, sur un filetage 16 de l'extrémité libre de l'axe 17 définissant la zone de retenue inférieure Z₁, entre la face inférieure 15a de l'insert 15 et la platine 10, la zone de retenue supérieure Z₂ étant quant à elle formée sur l'insert 15 entre deux rebords radiaux 18,19 constituant les épaulements d'arrêt du ligament.

La figure 10 illustre un développement de la variante de réalisation de la figure 8, selon lequel un second insert 20, enfile sur la moitié haute de l'axe 13, est en appui sur le gradin 11 du premier insert 12. Ceci permet, en cas de besoin, d'augmenter le diamètre de la zone de retenue supérieure Z₂ initialement definie par l'axe 13.

Dans le deuxième mode de réalisation de l'invention représenté sur la figure 11, la tête 2c de l'implant est également réalisée à partir d'un insert cylindrique 21, enfilé par un perçage central 23 sur un axe 24 saillant au-delà d'un gradin 40 formé sensiblement à mi-hauteur d'une partie fixe de la tête, représentée seule sur la figure 13. Ici toutefois, l'insert 21 repose sur le gradin 40 par une base 25 bombée sous une forme sphérique, ménagée au-delà d'un épaulement 22 regardant l'extrémité libre de la tête. Par ailleurs, et comme le montre mieux la figure 12, son perçage central 23 s'effile, depuis la face supérieure de l'insert 21, sous la forme d'une première partie tronconique 23a, sensiblement jusqu'à hauteur de l'épaulement 22 où, présentant un diamètre à peine supérieur à celui de l'axe 24, il s'élargit à nouveau sous la forme d'une seconde partie tronconique 23b débouchant sur la face inférieure bombée de la base 25.

Ainsi, une fois en appui sur le gradin 40 par sa base sphérique 25, l'insert 21 dispose d'une liberté de pivotement autour du centre de l'orifice 26 de jonction des deux parties tronconiques 23a,23b de son perçage central 23. Il s'ensuit qu'un ligament, tel que L₁, engagé autour de la zone de retenue supérieure Z₂ de la tête 2c, définie sur la surface latérale de l'insert 21, et arrêté par l'épaulement 22, pourra librement s'orienter en fonction des mouvements de flexion du rachis. Le tronçon cylindrique inférieur de grand diamètre 27 de la tête 2c pourra en cas de besoin être doté d'un rebord radial autour du gradin 40, pour définir une seconde zone de retenue.

Sur les figures 11 et 13, ainsi que sur les figures 4 à 8 et 10, on notera encore la présence, sur l'extrémité libre de la tête extra-osseuse ou de l'axe 13, d'une excroissance filetée 28, sur laquelle se visse la coiffe amovible 7 décrite en référence à la figure 2.

La figure 13 illustre une tête d'implant 2d conforme à un troisième mode de réalisation de l'invention, dans lequel un insert 29, dont la surface cylindrique forme une zone de retenue de ligament Z₂, entre des rebords radiaux supérieur 30 et inférieur 31, coiffe librement, par une cavité intérieure sphérique 32, une rotule 33, formée à l'extrémité d'un col 34 saillant sur une platine 41 de la tête. L'insert 29, réalisé en un alliage de titane à mémoire de forme, s'emboîte sur la rotule 33 grâce à quatre fentes axiales 35 pratiquées dans la paroi de la cavité 32, à 90° l'une de l'autre, comme représenté sur la figure 15. Un second insert 36, en forme de U, dont la surface extérieure est délimitée par des rebords périphériques supérieur 37 et inférieur 38, embrasse en outre le col 34 de la rotule 33.

Dans ce mode de réalisation de l'invention, les deux inserts 29 et 36 peuvent chacun recevoir, entre leurs rebords 30,31 ou 37,38, l'extrémité d'un ligament respectif L₁ ou L₂ et, pouvant pivoter librement, le premier autour de la rotule 33 et le second autour du col 34, ils permettent aux deux ligaments de s'orienter à volonté en fonction des flexions du rachis, sans venir en contact mutuel, comme on le voit sur la figure 14.

## Revendications

1. Implant chirurgical pour stabilisateur intervertébral (1a 1b 1c 1d) formé d'une tige intraosseuse (3) qui, d'un côté, comporte une extrémité filetée (4) d'implantation dans l'os et, de l'autre, se prolonge par une tête extra-osseuse cylindrique à section circulaire (2 ; 2b ; 2c ; 2d) susceptible de soutenir au moins deux ligaments souples (L1, L2 L3) par l'une de leurs extrémités en forme de boucle, caractérisé en ce que la tête extra-osseuse (2 ; 2b ; 2c ; 2d) présente au moins deux zones de retenue de ligament (Z1, Z2) définies sur les surfaces périphériques de tronçons respectifs étagés de ladite tête extra-osseuse, chacune de ces zones de retenue étant délimitée, au moins à son extrémité la plus proche de la tige intra-osseuse, par un épaulement annulaire (9, 6) constituant une butée axiale pour un ligament (L1, L2, L3).

2. Implant selon la revendication 1, caractérisé en ce que, une zone de retenue de ligament (Z1) étant directement contiguë à la tige intra-osseuse (3), l'épaulement (6) qui la délimite à son extrémité adjacente à cette dernière est défini, au moins partiellement, par une collerette radiale (5) formée à hauteur de la jonction entre la tête extra-osseuse (2 ; 2b ; 2c ; 2d) et la tige intra-osseuse (3).

3. Implant selon la revendication 2, caractérisé en ce que ladite collerette se prolonge sur une certaine hauteur le long de la tige (3), en direction de l'extrémité d'implantation (4) de cette dernière.

4. Implant selon l'une quelconque des revendications 1 à 3, caractérisé en ce que chacune des zones de retenue (Z1 ou Z2) formées sur la tête extra-osseuse (2 ; 2b ; 2c ; 2d) est délimitée, à son extrémité adjacente à la seconde (Z2 ou Z1) par un épaulement regardant la seconde extrémité de ladite zone de retenue.

5. Implant selon la revendication 4, caractérisé en ce que la tête extra-osseuse (2) étant monobloc, les deux épaulements sont respectivement définis par les faces opposées d'une collerette radiale (9) de celle-ci.

6. Implant selon l'une quelconque des revendications 1 à 4, caractérisé en ce que l'un au moins des tronçons de la tête extra-osseuse (2b) définissant les zones de retenue (Z1, Z2) est formé par un insert (12 ; 15 ; 20) enfilé et immobilisé axialement sur un axe (13 ; 17) solidaire de la tige (3).

7. Implant selon la revendication 6, caractérisé en ce que l'épaulement délimitant la zone de retenue (Z1, Z2) formée sur l'insert (12 ; 15), à l'une au moins de ses extrémités, est défini, au moins partiellement, par un rebord supérieur et un rebord inférieur radiaux (14 ; 19) dudit insert.

8. Implant selon l'une quelconque des revendications 1 à 7, caractérisé en ce que l'un au moins des tronçons de la tête extra-osseuse définissant les zones de retenue présente une forme tronconique.

9. Implant selon l'une quelconque des revendications 1 à 4, caractérisé en ce que :
- l'un au moins des tronçons de la tête extra-osseuse (2c), définissant les zones de retenue (Z1, Z2) est formé par un insert (21) enfilé, par un perçage central (23), sur un axe (24) solidaire de la tige (3) et reposant sur un gradin (40) de la tête (2c) par une base bombée sphérique (25) formée sous l'épaulement (22) de la zone de retenue (Z2) qu'il définit, le perçage central (23) de l'insert (21) étant formé de deux parties (23a, 23b) s'évasant chacune vers une face d'extrémité respective de l'insert, et communiquant entre elles par un orifice de jonction (26) ayant un diamètre à peine supérieur à celui de l'axe (24) sur leque est enfilé l'insert (21);
- le tronçon cylindrique inférieur de grand diamétre (27) de la tête extra-osseuse (2c) peut être doté d'un rebord radial autour du gradin (40) pour définir une seconde zone de retenue ;
- l'extrémité libre de la tête extra-osseuse ou de l'axe (13 ; 24) est pourvue d'une excroissance filetée (28) sur laquelle se visse une coiffe (7).

10. Implant selon l'une quelconque des revendications 1 à 4, caractérisé en ce que :
- la tête extra-osseuse (2d) comporte, à l'extrémité d'un col (34) solidaire de la tige (3), une rotule (33) sur laquelle est emboîté, avec liberté de pivotement, un insert cylindrique (29) constituant l'un desdits tronçons de la tête extra-osseuse, dont la surface périphérique délimite une zone de retenue respective (Z2) de ligament entre deux épaulements formés par des rebords radiaux supérieur (30) et inférieur (31) des faces d'extrémité de l'insert (29);
- l'insert (29) coiffe librement, par une cavité intérieure sphérique (32), une rotule (33), formée à l'extrémité d'un col (34) saillant sur une platine (41) de la tête (2d);
- l'insert (29) s'emboîte sur la rotule (33) par quatre fentes axiales (35) pratiquées dans la paroi de la cavité (32) à 90° l'une de l'autre.

11. Implant selon la revendication 10, caractérisé en ce qu'un second tronçon de la tête extra-osseuse (2d) est constitué par un insert en forme de U (36) qui embrasse librement le col (34) de la rotule (33), la surface périphérique extérieure dudit insert en forme de U étant délimitée par deux rebords périphériques supérieur (37) et inférieur (38), pour former la zone de retenue de ligament correspondante.

## Claims

1. Surgical implant for an intervertebral stabilizer (1a, 1b, 1c, 1d) formed from intro-osseous rod (3) which, on one side, has an end (4) for implanting in the bone and on the other is extended by a cylindrical extra-osseous head with circular cross section (2, 2b, 2c, 2d) able to peripherally retain at least two flexible ligaments (L1, L2, L3) by one of their loop-shaped ends, characterized in that the extra-osseous head (2, 2b, 2c, 2d) has at least two ligament retaining zones (Z1, Z2) defined on the peripheral surfaces of respective stepped portions of said extra-osseous head, each of there retaining zones being defined, at least at its end closest to the intra-osseous rod, by a shoulder (9, 6) constituting an axial abutment for a ligament (L1, L2, L3).

2. Implant according to claim 1, characterized in that, as a ligament retaining zone (Z1) is directly contiguous with the intra-osseous rod (3), the shoulder (6) which defines it at its end adjacent to the latter is at least partly defined by a radial collar (5) formed level whith the junction between the extra-osseous head (2, 2b, 2c, 2d) and the intra-osseous rod (3).

3. Implant according to claim 2, characterized in that, said collar (5) is extending over a certain height along the rod (3), in direction of the implantation end (4) of the latter.

4. Implant according to any of the preceding claims, characterized in that each of the retaining zones (Z1 or Z2) formed on the extra-osseous head (2, 2b, 2c, 2d) is defined, at its end adjacent to the second (Z2 or Z1) by a shoulder facing the second end of said retaining zone.

5. Implant according to claim 4, characterized in that as the extra-osseous head (2) is in one piece, the two shoulders are respectively defined by the opposite faces of a radial collar (9) thererof.

6. Implant acording to any of the claims 1-4, characterised in that at least one of the portions of the extra-osseous head (2b) defining the retaining zones (Z1, Z2) is formed by a threaded insert (12, 15, 20) axially immobilized on a spindle (13, 17) integral with the rod (3).

7. Implant acording to claim 6, characterized in that the shoulder defining the retaining zone (Z1, Z2) formed on the insert (12, 15), at at least one of its ends, is at least partly defined by a radial flange (14, 19) of said insert.

8. Implant according to any of the preceding claims, characterized in that at least one of the portions of the extra-osseous head defining the retaining zones has a truncated cone shape.

9. Implant acording to any of the claims 1-4, characterized in that
- at least one of the portions of the extra-osseous head (2c) defining the retaining zones (Z1, Z2) is formed by an insert (21) threaded by a central opening (23) onto a spindle (24) integral with the rod (3) and resting on a step (40) of the head (2c) by a curved base (25) formed beneath the shoulder (22) of the retaining zone (Z2) which it defines, the central opening (23) of the insert (21) being formed from two portions (23a, 23b) each widening towards a respective end face of the insert and communicating with one another by an orifice (26) having a diameter which is only just larger that of the spindle (24) onto which is threaded the insert (21);
- the large diameter, lower cylindrical portion (27) of the head (2c) can, if need be, be given a radial flange around the step (40) in order to define a second retaining zone;
- the free end of the extra-osseous head or spindle (13, 24) has a threated excrescence (28), onto which is screwed a detachable cap (7).

10. Implant according to any of the claims 1-4, characterized in that
- the extra-osseous head (2d), has at the end of a neck (34) integral with the rod (3), a knee joint (33), onto which is fitted, with a freedom of pivoting, a cylindrical insert (29) constituting one of the said portions of the extra-osseous head, whose peripheral surface defines a respective retaining zone (Z2) of the ligament between two shoulders formed by projecting upper (30) and lower (31) flanges of the end faces of the insert (29);
- the insert (29) freely covers by an inner spherical cavity (32), a knee joint (33) formed at the end of a neck (34) projecting over a plate (41) of the head (2d);
- the insert (29) is fined onto the knee joint (33) by means of four axial slots (35) made in the wall of the cavity (32) at 90° from one another.

11. Implant according to claim 10, characterized in that a second portion of the extra-osseous head (2d) is constitued by a U-shaped insert (36), which freely embraces the neck (34) of the knee joint (33), the outer peripheral surface of said U-shaped insert being defined by upper (37) and lower (38) peripheral flanges in order to form the corresponding ligament retaining zone.

## Patentansprüche

1. Implantat für Knochenchirugie für Zwischenwirbelstabilisator (1a, 1b, 1c, 1d), bestehend aus einem intraossalen Stift (3), der an einem der beiden Enden, das zur Implantation im Knochen bestimmt ist, ein Gewinde (4) aufweist, während das andere Ende, das durch einen zylinderförmigen, extraossalen Kopf mit kreisförmigem Querschnitt (2, 2b, 2c, 2d) verlängert wird, dazu bestimmt ist, mindestens zwei biegsame Ligamente (L1, L2, L3) an einem ihrer schleifenförmigen Enden zu stützen, und dadurch gekennzeichnet, daß das extraossale Kopfende (2, 2b, 2c, 2d) mindestens zwei Bereiche (Z1, Z2) aufweist, die ein Ligament stützen können, und die von den peripheren Oberflächen der jeweiligen, übereinanderliegenden Abschnitte des erwähnten extraossalen Kopfendes gebildet werden, wobei jeder dieser Stützbereiche zumindest an dem dem intraossalen Stift zunächst liegenden Ende durch einen ringförmigen Bund (9, 6) begrenzt wird, der einem Ligament (L1, L2, L3) einen axialen Halt bietet.

2. Implantat gemäß Patentanspruch 1, dadurch gekennzeichnet, daß bei einem Stützbereich des Ligaments (Z1), der direkt neben dem intraossalen Stift (3) liegt, der Bund (6), der diesen Stützbereich an dem Ende begrenzt, das direkt neben dem intraossalen Stift liegt, zumindest teilweise durch einen radialen Ring (5) gebildet wird, der aus der Verbindungsstelle zwischen dem extraossalen Kopfende (2, 2b, 2c, 2d) und dem intraossalen Stift (3) entsteht.

3. Implantat gemäß Patentanspruch 2, dadurch gekennzeichnet, daß der erwähnte Bund sich längs des Stiftes (3) über eine gewisse Höhe in Richtung des implantierten Endes (4) dieses Stiftes erstreckt.

4. Implantat gemäß einem der Patentansprüche 1 bis 3, dadurch gekennzeichnet, daß jeder der Stützbereiche (Z1 bzw. Z2), die auf dem extraossalen Kopfende (2, 2b, 2c, 2d) ausgeformt sind, an dem Ende, das neben dem zweiten (Z2 bzw. Z1) liegt, durch einen Bund begrenzt wird, der zum anderen Ende des erwähnten Stützbereichs weist.

5. Implantat gemäß Patentanspruch 4, dadurch gekennzeichnet, daß die beiden Bünde jeweils durch die entgegengesetzten Seiten eines radialen Ringes (9) des extraossalen Kopfendes (2) gebildet werden, da dieses aus einem Stück besteht.

6. Implantat gemäß einem der Patentansprüche 1 bis 4, dadurch gekennzeichnet, daß mindestens einer der Abschnitte des extraossalen Kopfendes (2b), die die Stützbereiche (Z1, Z2) bilden, von einem Einsatz (12, 15, 20) gebildet wird, der auf eine Achse (13, 17), die fest mit dem Stift (3) verbunden ist, geschoben und axial befestigt wird.

7. Implantat gemäß Patentanspruch 6, dadurch gekennzeichnet, daß der Bund, der den Stützbereich (Z1, Z2) begrenzt, der auf dem Einsatz (12, 15) an mindestens einem seiner Enden ausgeformt ist, zumindest teilweise durch einen oberen und einen unteren radialen Rand (14, 19) des erwähnten Einsatzes gebildet wird.

8. Implantat gemäß einem der Patentansprüche 1 bis 7, dadurch gekennzeichnet, daß mindestens einer der Abschnitte des extraossalen Kopfendes, die die Stützbereiche begrenzen, die Form eines Kegelstumpfes hat.

9. Implantat gemäß einem der Patentansprüche 1 bis 4, dadurch gekennzeichnet, daß:
- mindestens einer der Abschnitte des extraossalen Kopfendes (2c), die die Stützbereiche (Z1, Z2) bilden, von einem Einsatz (21) gebildet wird, der durch eine zentrale Bohrung (23) auf eine Achse (24) geschoben wird, die fest mit dem Stift (3) verbunden ist und der mit seinem kugelförmigen, gewölbten unteren Ende (25), das unter dem von ihm begrenzten Bund (22) des Stützbereichs (52) auf einem Absatz (40) des Kopfendes (2c) ruht, wobei die zentrale Bohrung (23) des Einsatzes (21) aus zwei Abschnitten (23a, 23b) besteht, die zum jeweiligen Ende des Einsatzes hin breiter werden und miteinander durch eine Verbindungsöffnung (26) verbunden sind, deren Durchmesser kaum größer als der der Achse (24) ist, auf die der Einsatz (21) geschoben wird;
- der Absatz (40) des zylinderförmigen, unteren Abschnitts mit großem Durchmesser (27) des extraossalen Kopfendes (2c) mit einem radialen Rand versehen werden kann, um einen zweiten Stützbereich zu bilden;
- das freie Ende des extraossalen Kopfendes bzw. der Achse (13, 24) eine Ausbuchtung mit Gewinde (28) aufweist, auf die ein Deckel (7) geschraubt wird.

10. Implantat gemäß einem der Patentansprüche 1 bis 4, dadurch gekennzeichnet, daß:
- das extraossale Kopfende (2d) am Ende eines Halses (34), der fest mit dem Stift (3) verbunden ist, ein Kugelgelenk (33) aufweist, auf dem ein zylinderförmiger Einsatz (29) schwenkbar aufgesteckt ist, der einen der erwähnten Abschnitte des extraossalen Kopfendes bildet und dessen periphere Oberfläche jeweils einen Stützbereich (52) für ein Ligament zwischen den oberen (30) und unteren (31) radialen Bünden der Enden des Einsatzes (29) abgrenzt;
- der Einsatz (29) durch einen kugelförmigen inneren Hohlraum (32) frei auf einem Gelenk (33) sitzt, das auf das Ende eines Halses (34) aufgesteckt wird, der wiederum auf einer Platte (41) des Kopfendes (2d) sitzt;
- der Einsatz (29) durch vier axiale Schlitze (35), die an der Wand des Hohlraums (32) im 90°-Abstand voneinander angebracht wurden, auf das Gelenk (33) paßt.

11. Implantat gemäß Patentanspruch 10, dadurch gekennzeichnet, daß ein zweiter Abschnitt des extraossalen Kopfendes (2d) von einem U-förmigen Einsatz (36) gebildet wird, der den Hals (34) des Gelenks (33) frei umschließt, wobei die periphere Oberfläche des erwähnten U-förmigen Einsatzes von zwei peripheren Rändern, einem oberen (37) und einem unteren (38) begrenzt wird, um so den Stützbereich des dazugehörigen Ligaments zu bilden.
